# EUROPÄISCHE PATENTSCHRIFT

(11) **EP 0 528 360 B1**
(45) Veröffentlichungstag und Bekanntmachung des Hinweises auf die Patenterteilung: **02.05.2002**
(21) Anmeldenummer: 92113816.0
(22) Anmeldetag: 13.08.1992
(51) Int. Cl.: C12N 15/00

(54) **Transgene Ratten, die in ihrem Genom mindestens ein menschliches Gen enthalten, das an der Blutdruckregulation beteiligt ist**
Transgenic rats having at least one human gene in their genome, participating in the regulation of the blood-pressure
Rats transgéniques qui ont dans leur génome au moins un gène humain participant à la régulation de la tension artérielle

(30) Priorität: 14.08.1991 DE 4126968
(43) Veröffentlichungstag der Anmeldung: 24.02.1993
(73) Patentinhaber: Ganten, Detlev, Prof. Dr. med., D-13125 Berlin (DE)
(72) Erfinder: Ganten, Detlev, Prof.Dr.med., O-13125 Berlin (DE); Mullins, John, Ph.D., Centre for Animal Genome, England EH9 3JQ (GB); Murakami, Kazuo, Prof., The University of Tsukuba, Ibaraki-ken, 305 (JP)

(56) Entgegenhaltungen:
- EMBASE (EXCERPTA MEDICA) DATABASE, ZUSAMMENFASSUNG NO. 91300079, AMSTERDAM, NL A. FUKAMIZU ET AL. 'Generation of transgenic mice with human renin and angiotensinogen genes' & Japanese Heart Journal 1991, Band 32, no. 4, Seite 553
- HYPERTENSION Bd. 17, Nr. 6, Juni 1991, Seiten 843 - 855 D. GANTEN ET AL. 'Transgenics rats: new animal models in hypertension research'
- PROCEEDINGS OF THE NATIONAL ACADEMY OF SCIENCES OF USA. Bd. 87, Nr. 13, Juli 1990, WASHINGTON US Seiten 5153 - 5157 H. OHKUBO ET AL. 'Generation of transgenic mice with elevated blood pressure by introduction of the rat renin and angiotensinogen genes'
- BIOCHEMICAL AND BIOPHYSICAL RESEARCH COMMUNICATIONS Bd. 165, Nr. 2, 15. Dezember 1989, NEW YORK, US Seiten 826 - 832 A. FUKAMIZU ET AL. 'Tissue-specific expression of the human renin gene in transgenic mice'
- NATURE Bd. 344, Nr. 6266, 5. April 1990, LONDON GB Seiten 541 - 544 J.J. MULLINS ET AL. 'Fulminant hypertension in transgenic rats harbouring the mouse Ren-2 gene'
- PROCEEDINGS OF THE NATIONAL ACADEMY OF SCIENCES OF USA. Bd. 81, Nr. 19, Oktober 1984, WASHINGTON US Seiten 5999 - 6003 H. MIYAZAKI ET AL. 'Structure of the human renin gene'
- BIOCHEMISTRY. Bd. 23, Nr. 16, 31. Juli 1984, EASTON, PA US Seiten 3603 - 3609 R. KAGEYAMA ET AL. 'Primary structure of human preangiotensinogen deduced from the cloned cDNA sequence'
- PROCEEDINGS OF THE NATIONAL ACADEMY OF SCIENCES OF USA. Bd. 89, Nr. 16, August 1992, WASHINGTON US Seiten 7806 - 7810 D. GANTEN ET AL. 'Species specificity of renin kinetics in transgenic rats harboring the human renin and angiotensinogen genes'

## Beschreibung

Die Erfindung betrifft transgene Ratten, in deren Genom mindestens ein menschliches Gen, dessen Genprodukt an der Blutdruckregulation beteiligt ist, integriert ist.
Die Erfindung betrifft ferner transgene Ratten und deren Nachkommen, die erhöhten Blutdruck (>90/>140 mm Hg) oder Bluthochdruck (>95/>160 mm Hg) aufweisen.
Schließlich betrifft die Erfindung Verfahren zur Herstellung der erfindungsgemäßen transgenen Ratten sowie deren Nachkommen und deren Verwendung für pharmakologische Untersuchungen.

Transgene Tiere sind genetisch veränderte Tiere, bei denen mindestens ein fremdes Gen in das Genom eingeschleust wurde. Mit diesen Tieren lassen sich Regulationsprozesse auf zellulärer Ebene in mit anderen Testsystemen nicht erreichter systematischer und spezifischer Weise untersuchen und beeinflussen. Darüber hinaus besteht die Möglichkeit, die Wirkung bestimmter Pharmaka an transgenen Tieren zu erproben und so die Entwicklung neuer pharmakologisch wirksamer Substanzen auszutesten.

Bei der zuerst entwickelten und am weitesten verbreiteten Technik zur Erzeugung transgener Tiere werden zunächst befruchtete Eizellen aus weiblichen Tieren entnommen. Anschließend wird die gewünschte Fremd-DNA (das "Transgen") in die Eizellen eingeschleust. Dazu stehen mehrere prinzipiell verschiedene Ansätze zur Verfügung: Einerseits können die Transgene über einen geeigneten retroviralen Vektor, andererseits durch Mikroinjektion in die Eizellen und damit in das Genom eingebracht werden (Palmiter R.D., Brinster R.L.: Germline transformation of mice. Ann.Rev.Genet. 20 (1986), 465-499). Rekombinante retrovirale Vektoren werden nach im Stand der Technik bekannten Verfahren in die Eizellen eingeschleust (Jaenisch R.: Transgenic animals. Science 240 (1988), 1468-1475). Bei der Mikroinjektion wird die clonierte Fremd-DNA vor der Fusion der Pronuclei von Sperma und Eizellen direkt in einen der beiden Pronuclei einer befruchteten Eizelle injiziert. Die Eizellen werden anschließend pseudoschwangeren Weibchen zum Austragen reimplantiert.

Transgene können auch in embryonale, fetale oder adulte pluripotente Stammzellen eingeführt werden (Capecchi M.: Altering the genome by homologous recombination. Science 244 (1991) 1288-1292). Embryonale Stammzellen können aus in vitro kultivierten Blastozysten isoliert und über viele Zellgenerationen stabil, d.h. ohne weitere Differenzierung in Kultur gehalten werden. Fremde DNA kann beispielsweise durch Elektroporation in die embryonalen Stammzellen eingeführt werden. Nach Selektion von Stammzellen, die die gewünschte fremde DNA tragen, werden diese in die innere Zellmasse von Blastozysten injiziert. Anschließend werden die Blastozysten in pseudoschwangere Weibchen reimplantiert. Da nicht alle Zellen der inneren Zellmasse der Blastozysten die Transgene tragen, ist das sich daraus entwickelnde Tier in bezug auf die Transgene eine Chimäre. Aufgrund der Pluripotenz der Stammzellen können alle Gewebearten und damit auch die Keimzellen die Transgene tragen. Durch nachfolgende Kreuzungen der Chimären erhält man Tiere, in denen alle Zellen die Transgene tragen. Die konventionelle Mikroinjektionstechnik befruchteter Eizellen und der Weg über embryonale Stammzellen zur Herstellung transgener Tiere haben sowohl praktische und methodische Vor- als auch Nachteile. Die Wahl der einzusetzenden Technik hängt in erster Linie von der experimentellen Zielsetzung und von den technischen Möglichkeiten des jeweiligen Labors ab.

Der besondere Vorteil der Mikroinjektionstechnik liegt darin, daß die Methode gut etabliert und relativ einfach in der Durchführung ist. Bei der Wahl geeigneter genomischer Genkonstrukte mit den entsprechenden Promotoren kann eine zielgerichtete Genexpression erreicht werden. Im allgemeinen ist aber damit zu rechnen, daß sowohl das endogene Gen als auch das Transgen exprimiert werden. Dementsprechend können Interferenzen zwischen beiden Genprodukten auftreten, die eine Auswertung der Versuchsergebnisse erschweren. Ein weiterer Vorteil der Mikroinjektionstechnik liegt darin, daß es relativ einfach ist, mehrere Gene gleichzeitig zu injizieren. Damit kann gegebenenfalls die Gesamtexpression der Genprodukte erhöht und die Ausprägung des Phänotyps verbessert werden.

Der Vorteil der embryonalen Stammzell-Technik ist vor allem darin zu sehen, daß die mit dem Transgen transfizierten Zellen vor der Reimplantation auf die Integration und die Wirkung der Transgene getestet werden können. So kann in Abgrenzung zur konventionellen Mikroinjektionstechnik das entsprechende endogene Gen durch homologe Rekombination mit dem Transgen aus einem Chromosom entfernt werden. Durch anschließende Kreuzungsexperimente lassen sich Tiere züchten, die das Transgen auf beiden Chromosomen tragen. Wenn in die Transgene Mutationen eingeführt werden, die die Expression des normalen Genproduktes verhindern, können mit dieser Technik die endogenen Gene ausgeschaltet und damit Funktionsstudien durchgeführt werden.

Die oben erläuterten Verfahren zur Erzeugung transgener Tiere in Kombination mit konventionellen gentechnologischen Verfahren liefern ungeahnte neue Möglichkeiten zur Schaffung von in vivo Tiermodellen zur Erkennung von Krankheitsprozessen und darauf aufbauend zur Entwicklung neuer Medikamente. Für diese Entwicklung einer neuen Generation kausal wirkender Pharmaka müssen zwei Grundvoraussetzungen gegeben sein:
1) die pathophysiologischen Ursachen der zu behandelnden Krankheiten müssen bekannt sein; und
2) es muß experimentelle Modelle geben, die die Prüfung der therapeutischen Wirksamkeit und der Spezifität von Substanzen in möglichst einfacher Weise und mit möglichst hoher Aussagekraft erlauben.

Einer der Krankheitsprozesse, die mit solchen Tiermodellen genauer erforscht werden können, ist der, welcher zu erhöhtem Blutdruck (d.h. zu Werten, die über dem Normbereich von 90 mm Hg diastolisch und 140 mm Hg systolisch liegen) und zu Bluthochdruck (Werte über 95 mm Hg diastolisch und 160 mm Hg systolisch) führt.

Bluthochdruck (Hypertonie) wird beim Menschen in primäre und sekundäre Formen unterteilt. Sekundäre Hypertonieformen haben eine bekannte Ursache, machen aber an der Gesamtzahl der Hypertoniker nur einen geringen Prozentsatz aus. Dagegen handelt es sich bei der primären Hypertonie um eine weitverbreitete Krankheit, an der 20 % der erwachsenen Bevölkerung leiden. Ihre Ursache ist unbekannt, wobei aber eine erbliche Komponente angenommen wird, die aus dem Zusammenspiel verschiedener an der Blutdruckregulation beteiligter Gene resultiert. Die derzeit verfügbaren Therapien sind daher nicht kausal, sondern weitgehend symptomatisch und mit zahlreichen Nebenwirkungen behaftet.

Bei der Regulation des Blutdrucks kommt dem Renin-Angiotensin-System (RAS) die entscheidende Funktion zu neben dem zentralen Nervensystem, der Niere und Hormonen wie Vasopressin (AVP), atrialer natriuretischer Faktor (ANP) oder Aldosteron. Vermittels des Effektorpeptids Angiotensin II ist das RAS an der Regulation des peripheren Gefäßwiderstandes und der Aufrechterhaltung der Elektrolyt- und Flüssigkeitshomöostase beteiligt. Ihm wird die kausale Bedeutung für die Genese verschiedener Formen des renalen Bluthochdruckes beigemessen, eine pathogenetische Beteiligung an der Entstehung der primären Hypertonie wird diskutiert (Ganten D., Ritz E., Lehrbuch der Hypertonie - Pathophysiologie, Klinik, Therapie, Epidemiologie, Stuttgart, New York: Schattauer Verlag, 1985).

Menschliches Angiotensinogen ist ein Hormon-Vorläufer-Molekül mit einem Molekulargewicht von 61 400. Es besteht aus einer einzelnen Polypeptidkette. Die DNA- und Aminosäuresequenz des Moleküls sind bekannt (Fukamizu et al., Biochem. Biophys. Res. Comm. 165 (1989), 826 - 832). Das zur Supergen-Familie der Protease-Inhibitor-Gene gehörende Angiotensinogen-Gen wird in der Leber exprimiert und das so erhaltene Angiotensinogen ins Blut abgegeben, wo es von dem Enzym Renin (EC 3,4,23,15.), einer Aspartylproteinase, gespalten wird. Renin (Miyazaki et al., Proc. Natl. Acad. Sci. USA 81 (1984), 5999 - 6003) wird von den glomerulären Zellen des juxtaglomerulären Apparates der Niere synthetisiert, gespeichert und sezerniert. Im Blut spaltet Renin Angiotensinogen in das N-terminale Dekapeptid Angiotensin I und in ein 450 Aminosäure-Polypeptid, Des-(Angiotensin I)-Angiotensinogen. Angiotensin I wird bei Kontakt mit dem Gefäßendothel durch das ubiquitär vorkommende Konversionsenzym (CE) in das physiologisch wirksame, multifunktionelle Oktapeptid Angiotensin II überführt.

Das zirkulierende Angiotensin II, Plasma-Angiotensin II, vermittelt über spezifische Rezeptoren eine Vielzahl physiologischer Effekte, wie Kontraktion der glatten Gefäßmuskulatur, Aldosteronfreisetzung in der Nebennierenrinde, direkte Natrium- und Wasserretention in der Niere und dadurch Blutdruckanstieg. Angiotensin II stimuliert ebenfalls den Salzappetit und Durst und hat bedeutende Effekte auf die Aktivität des autonomen Nervensystems und auf die Ausschüttung von Hypophysenhormonen. Neben der Bildung von Angiotensin II (ANG II) im Plasma (hormonales RAS) kommt der Bildung von Angiotensin II im Gewebe (Gewebe RAS, parakrines RAS) ebenfalls eine Bedeutung zu. Lokale Angiotensin II-Bildung wurde insbesondere im Gehirn, in der Nebenniere, im Herzen und in den Gefäßwänden nachgewiesen (Ganten D., Lindpaintner K., Ganten U., Peters J.: Transgenic rats: New animal models in hypertension research. Hypertension 17 (1991), 843-855. Lindpaintner K., Ganten D.: The cardiac renin-angiotensin system. An appraisal of present experimental and clinical evidence. Circ. Res. 4 (1991), 905-921. Schelling P., Fischer H., Ganten D.: Angiotensin and cell growth: A link to cardiovascular hypertrophy? J.Hypertens. 9 (1991), 3-15). Eine Beteiligung dieses Gewebe-Angiotensin II bei der Blutdruckregulation wurde in verschiedenen Studien gezeigt. Die blutdrucksenkende Wirkung von Hemmstoffen des RAS bei normalem oder sogar erniedrigtem Plasma-RAS spricht ebenfalls für die funktionelle Bedeutung lokaler Angiotensin-Synthese.

Der Abbau von Angiotensin II in inaktive Peptide erfolgt durch Angiotensinasen. Die Kontrolle der physiologischen Aktivität des Renin-Angiotensin-Systems erfolgt in erster Linie über die Steuerung der Sekretionsrate von Renin und damit die Bildungsgeschwindigkeit von Angiotensin II. Die kurze Plasma-Halbwertszeit von Renin und die rasche Ausschüttung aus Speichern ermöglichen eine schnelle Anpassung an sich ändernde physiologische und pathophysiologische Bedingungen. Den Regulationsmechanismen des Renins kommt damit eine herausragende Rolle bei der Steuerung des Blutdruckes zu.

Spezifischen Inhibitoren des Renins fällt daher eine Schlüsselrolle bei der kausalen Therapie der Hypertonie zu. Bisher können solche Entwicklungen fast ausschließlich in Primaten durchgeführt werden, da das Renin eine hohe Art-Spezifität aufweist, d.h. Renin-Inhibitoren, die für den Menschen entwickelt wurden, reagieren nur mit menschlichem oder Primaten-Renin, nicht aber mit Renin anderer Spezies. Hieraus ergeben sich viele Schwierigkeiten für die Entwicklung neuer blutdruckregulierender Substanzen, die mit konventionellen Methoden nur mit großem Aufwand überwunden werden können.

Zur weiteren Erforschung des RAS wurden bereits transgene Tiere, die Komponenten des RAS enthalten, erzeugt. Es handelt sich dabei um transgene Mäuse, in deren Genom das menschliche Renin-Gen (Fukamizu et al. a.a.O.), das Ratten-Renin-Gen und/oder das Ratten-Angiotensinogen-Gen (Ohkubo et al., Proc. Natl. Acad. Sci. USA 87 (1990), 5153 - 5157) eingeschleust wurde(n). Darüber hinaus sind transgene Ratten bekannt, die das Maus Ren 2-Renin-Gen exprimieren (Mullins et al., Nature 344 (1990), 541-544). Die Renin- und Angiotensinogen-Gene wurden in diesen Tieren gewebespezifisch exprimiert, sofern sie unter der Kontrolle des homologen Promotors standen. Fukamizu et al. haben keine Untersuchungen zur Auswirkung der transgenen Expression auf den Blutdruck durchgeführt. Die Arbeiten von Mullins et al. haben gezeigt, daß es trotz erniedrigter Renin-Werte im Plasma und in der Niere zu erhöhtem Blutdruck kommen kann. Eine eindeutige Zuordnung zwischen der Wirkung der Komponenten des RAS und dem Blutdruckanstieg konnte nicht aufgezeigt werden. Auch in dem von Ohkubo et al. gewählten System ist eine Interpretation der Daten schwierig und führt nicht zu eindeutigen Ergebnissen, da bekannt ist, daß Maus-Renin Ratten-Angiotensinogen, nicht aber Ratten-Renin Maus-Angiotensinogen spaltet.

Das ideale Modellsystem, in dem nur die Expressionsprodukte der Transgene miteinander reagieren und mit dem man dadurch einen unzweideutigen Kausalzusammenhang zwischen der Aktivität von Genprodukten, die an der Blutdruckregulierung beteiligt sind und der Änderung des Blutdrucks herstellen kann, ist im Stand der Technik nicht bekannt. Nur ein solches Modellsystem erfüllt aber die oben dargestellten Voraussetzungen zur Entwicklung einer neuen Generation kausal gegen Bluthochdruck wirkender Pharmaka. In einem bevorzugten solchem Modellsystem interagieren lediglich die Expressionsprodukte menschlicher Gene, deren Genprodukte an der Blutregulierung beteiligt sind, miteinander.

Der Erfindung liegt daher das technische Problem zugrunde, Modellsysteme bereitzustellen, die die Untersuchung der an der Blutdruckregulation des Menschen beteiligten Gene und Genprodukte in human-spezifischer Weise gestatten.

Die Lösung dieses technischen Problems wird durch die Bereitstellung der in den Patentansprüchen gekennzeichneten Ausführungsformen erzielt.

Gegenstand der Erfindung ist somit eine transgene Ratte, deren Genom zwei exprimierbare menschliche Gene enthält, deren Genprodukte an der Blutdruckregulation beteiligt ist. Vorzugsweise lassen sich mit der transgenen Ratte invasive chronische Instrumentierungen und hämodynamische sowie endokrinologische Analysen durchführen.
Der Begriff "transgen" bedeutet, daß eines oder mehrere zusätzliche Gene in das Genom integriert ist/sind.
Der Begriff "Ratte" betrifft zoologisch-systematisch betrachtet Tiere, die zur Gattung Rattus gehören.

Das erfindungsgemäße transgene Tiermodell wurde beispielhaft an der Art Rattus norvegicus (für die Gewinnung befruchteter Eizellen wurden Ratten der Laborstämme Sprague Dawley und Wistar Kyoto gepaart) entwickelt. Es kann jedoch auch mit anderen Arten der Gattung Rattus, z.B. der Art Rattus rattus erzeugt werden.

Der Begriff "exprimierbar" bedeutet, daß das Gen transkribiert und die so entstandene mRNA translatiert werden kann, wobei ein Genprodukt entsteht, das seine physiologische Funktion erfüllen kann. Ein exprimierbares Gen ist daher auch mit allen notwendigen Regulationssequenzen ausgestattet.

Unter dem Begriff "Genom" einer Ratte versteht man deren gesamte Erbinformation. Diese ist auf den Chromosomen aller Zellen, also der somatischen und der Keimzellen lokalisiert.

Der Begriff "Blutdruckregulation" betrifft das Einstellen des Blutdrucks auf den Normalwert. Dieser liegt beim Menschen und bei der Ratte bei 90 mm Hg diastolisch und 140 mmHg systolisch. Bei Werten von >90 und ≤95 mm Hg diastolisch und >140 und ≤160 mm Hg systolisch spricht man von "erhöhtem Blutdruck". "Bluthochdruck" wird nach Definition der WHO erreicht, wenn die Werte über 95 mm Hg diastolisch und über 160 mm Hg systolisch liegen.

Der Begriff "invasive chronische Instrumentierungen" bezeichnet die chronische Implantation von Kathetern in Arterien, Venen, Harnleiter und auch intrazerebroventrikulär. Hierdurch lassen sich sowohl exogene Substanzen infundieren, als auch gleichzeitig die hierdurch hervorgerufenen Wirkungen messen.

Der Begriff "hämodynamische sowie endokrinologische Analysen" betrifft die Messung hämodynamischer Parameter nach Katheterimplantation, die auf die Blutdruckregulation Einfluß haben, als auch die Bestimmung kardiovaskulärer und renaler Funktionswerte im Zusammenhang mit der Wirkung des Transgens (der Transgene) (hämodynamische Analyse) sowie die Gewinnung von Blut. zur Bestimmung der Wechselwirkungen zwischen dem Transgen (den Transgenen) bzw. dem (den) Genprodukt(en) und anderen an der Blutdruckregulation beteiligten Hormonsystemen (endokrinologische Analyse).

Das Genom der erfindungsgemäßen Ratte enthält die zwei exprimierbaren menschlichen Gene Renin und Angiotensinogen.

Bei der erfindungsgemäßen transgenen Ratte steht mindestens eines beiden der Gene unter der Kontrolle eines homologen Promotors.
Unter einem "homologen" Promotor versteht man einen Promotor, der die Expression des Gens unter natürlichen Bedingungen steuert.

Bei der erfindungsgemäßen transgenen Ratte steht mindestens eines der beiden Gene unter der Kontrolle eines heterologen Promotors.
Der Begriff "heterolog" bedeutet, daß das Gen nicht unter der Kontrolle seines natürlichen Promotors steht, sondern eines Promotors, der unter natürlichen Bedingungen die Expression eines anderen Gens steuert.

Mindestens eines der beiden menschlichen Gene der erfindungsgemäßen Ratte, dessen Genprodukt an der Blutdruckregulation beteiligt ist, steht unter der Kontrolle eines homologen Promotors. In der anderen Variante steht mindestens eines der menschlichen Gene unter der Kontrolle eines heterologen Promotors.

In der erfindungsgemäßen transgenen Ratte enthält das Genom mindestens ein Gen, das Renin codiert und mindestens ein Gen, das Angiotensinogen codiert.

Die erfindungsgemäße transgene Ratte weist einen durch das die eingeführten Gene erhöhten Blutdruck, vorzugsweise von größer als 9C mmHg diastolisch und 140 mm Hg systolisch oder einen dadurch verursachten Bluthochdruck, vorzugsweise von größer als 95 mm Hg diastolisch und 160 mm Hg systolisch auf.

Ein weiterer Gegenstand der Erfindung ist ein Verfahren zur Erzeugung einer das menschliche Renin- und das menschliche Angiotensinogen-Gen tragenden transgenen Ratte, bei die Gene spätestens im Achtzellenstadium in die Ratte eingeführt werden oder in ihre Vorfahren eingeschleust worden sind.

Unter "Achtzellenstadium" versteht man das Stadium nach dreimaliger Teilung der befruchteten Eizelle in der Entwicklung des Embryos. In diesem sogenannten Morulastadium besitzen noch alle 8 Zellen Pluripotenz, das heißt, sie können sich in alle Gewebetypen einschließlich der Keimzellen entwickeln. Nach einer weiteren Teilung, also im "Sechzehnzellstadium" ist diese Pluripotenz nicht mehr gegeben.

Ein weiterer Gegenstand der Erfindung ist die Verwendung der erfindungsgemäßen transgenen Ratte für pharmakologische Untersuchungen zur Blutdruckregulierung.
Beispiele für solche pharmakologischen Untersuchungen sind Untersuchungen, mit denen man den Einfluß von für menschliches Renin spezifischen Inhibitoren auf den Blutdruck der erfindungsgemäßen transgenen Ratten mißt. Dabei können die Ratten vor der Gabe des Inhibitors einer natriumarmen Diät ausgesetzt werden. In weiteren Beispielen für solche pharmakologischen Untersuchungen kann die Wirkung von Angiotensin-Rezeptor-Antagonisten, z.B. von DUP 753 auf den Blutdruck der erfindungsgemäßen transgenen Ratten ermittelt werden.

Die Figuren zeigen:

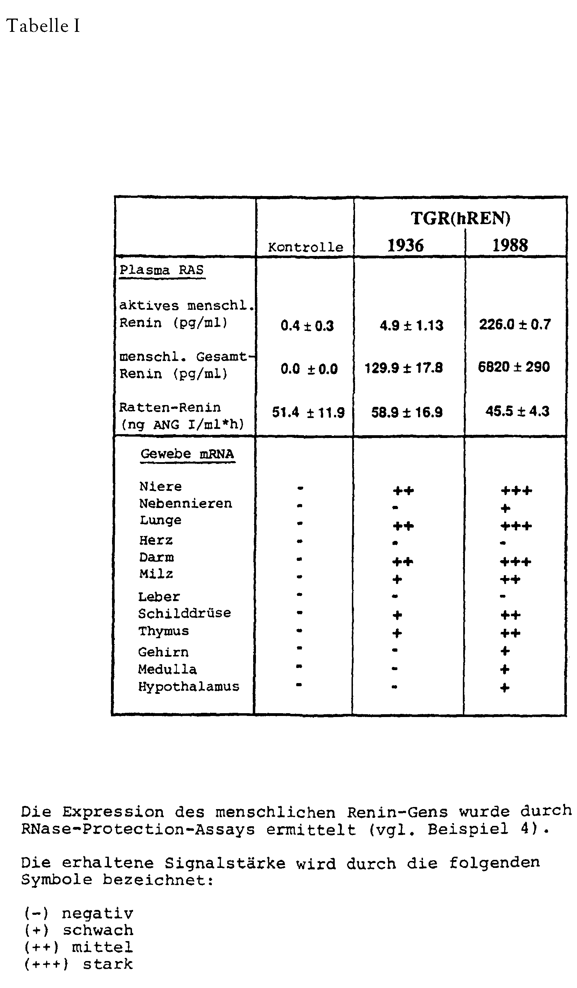

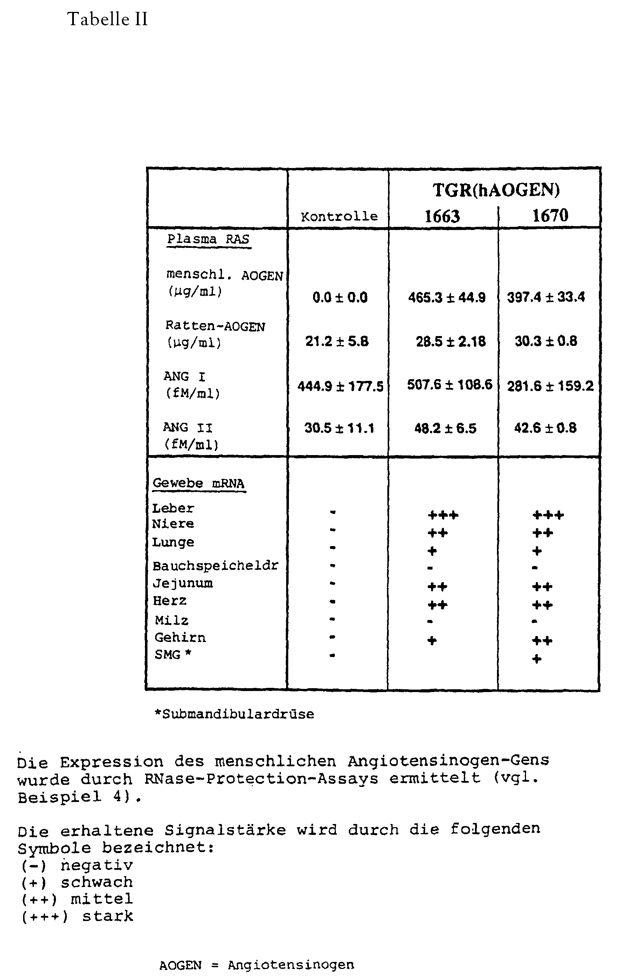

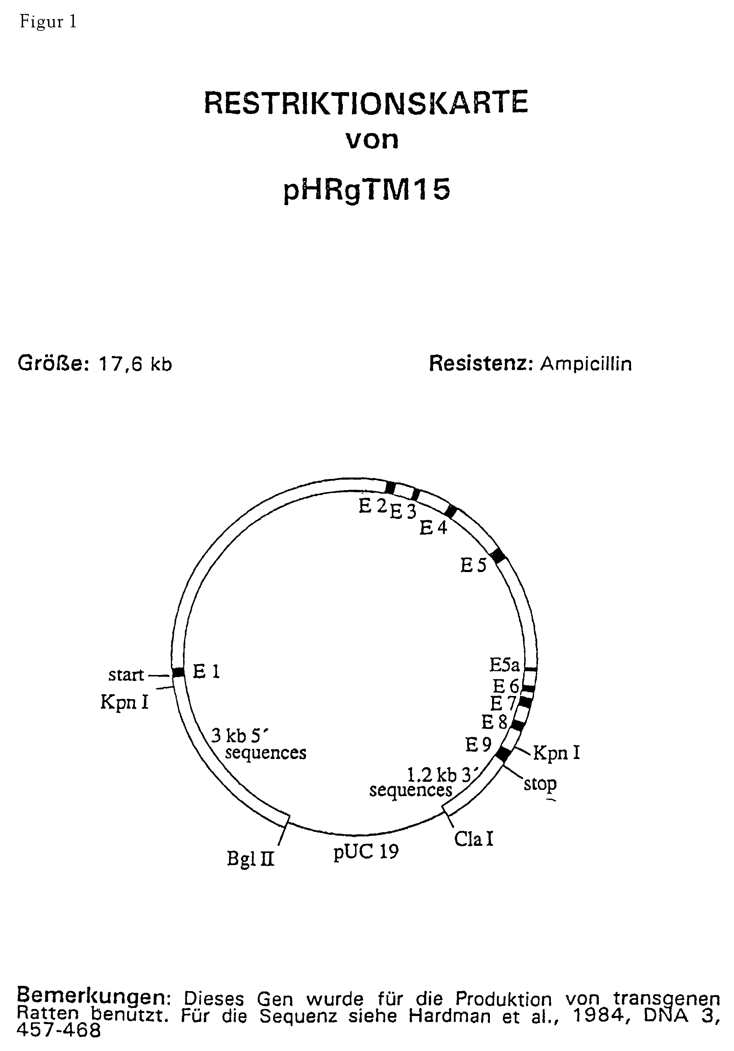

**Fig. 1: Restriktionskarte des Plasmids pHRgTM15**
   Plasmid pHRgTM15 enthält das gesamte menschliche Renin-Gen (10 Exons und 9 Introns) einschließlich 3 kb 5'-flankierender und 1,2 kb 3'-flankierender Sequenzen (dicke Linienführung) cloniert in den Plasmidvektor pUC19 (dünne Linienführung). pUC19 wurde für die Clonierung dergestalt modifiziert, daß die Enden nach Spaltung mit KpnI mit einem BglII-Linker und einem ClaI-Linker versehen wurden.

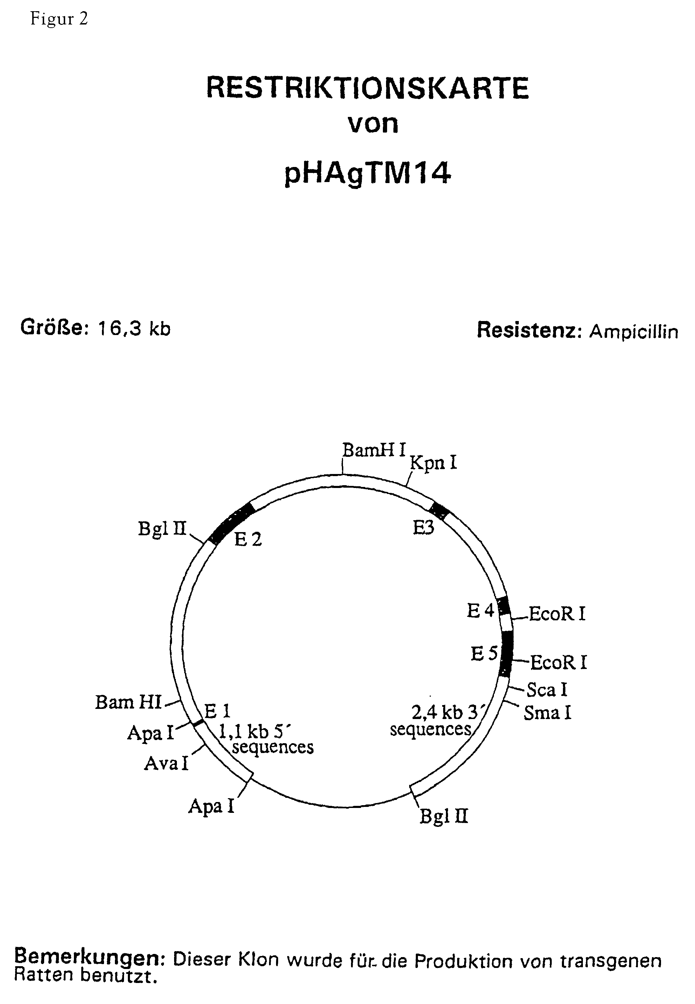

**Fig. 2: Restriktionskarte des Plasmids pHAgTM14**
   Plasmid pHAgTM14 enthält das gesamte menschliche Angiotensinogen-Gen (5 Exons und 4 Introns) einschließlich 1,1 kb 5'flankierender und 2,4 kb 3'-flankierender Sequenzen (dicke Linienführung) cloniert in den Plasmidvektor pUC19 (dünne Linienführung). pUC19 DNA wurde dazu mit BamHI gespalten und mit NheI-Linkern versehen.

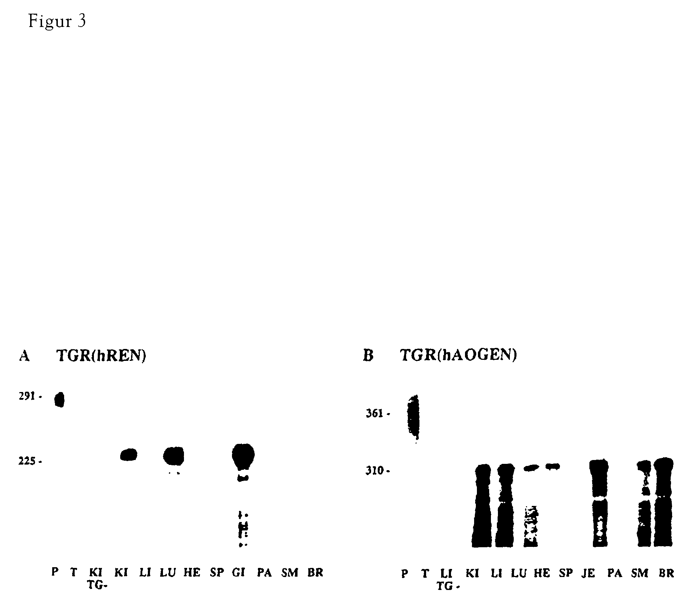

**Fig. 3: RNase-Protection-Assay-Analyse der Genexpression von menschlichem Renin (hREN) und menschlichem Angiotensinogen (hAOGEN) in transgenen Ratten**
   a) Expression von menschlicher Renin-mRNA in verschiedenen Geweben Renin-transgener Ratten.
      Abkürzungen: P: nicht gespaltene cDNA-Sonde für menschliches Renin (vgl. Beispiel 1); T: tRNA; KITG-: Rattennieren-RNA aus Transgen-negativen Geschwistern (negative Kontrolle); KI: Niere; LI: Leber; LU: Lunge; HE: Herz; SP: Milz; GI: Gastrointestinaltrakt; PA: Pankreas; SM: Unterkieferdrüse; BR: Gehirn.
      Für die Elektrophorese wurden in jede Tasche 50 µg Gesamt-RNA aufgetragen.
   b) Gewebespezifische Expression menschlicher Angiotensinogen-mRNA in Angiotensinogen-transgenen Ratten
      Abkürzung: vgl. unter a), oben; P: nicht gespaltene cDNA-Sonde für menschliches Angiotensinogen (vgl. Beispiel 2). Für die Elektrophorese wurden in jede Tasche 50 µg Gesamt-RNA aufgetragen, mit der Ausnahme, daß 5 µg Leber-Gesamt-RNA aufgetragen wurden.
      Als negative Kontrolle diente Rattenleber-RNA aus Transgen-negativen Geschwistern (LITG-).

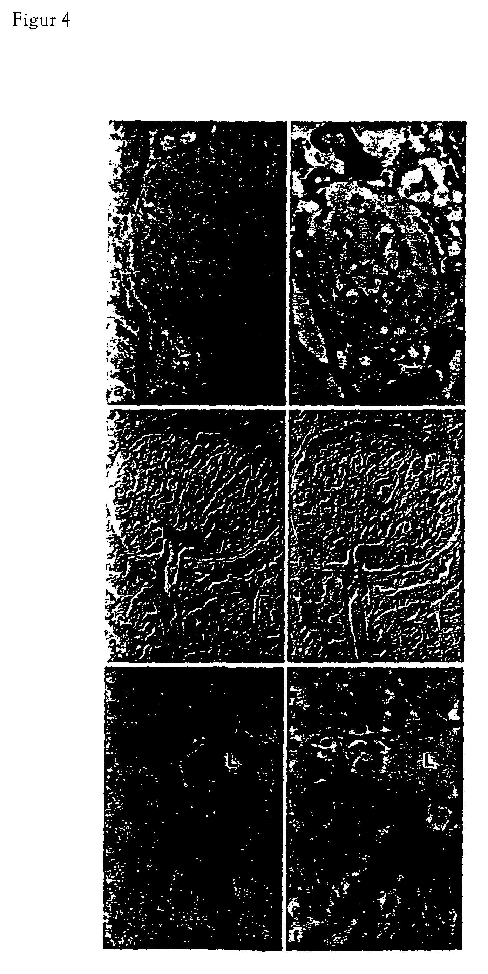

**Fig. 4: In situ-Hybridisierung und Immunhistochemie von Renin- und Angiotensinogen-Genprodukten in Geweben transgener Ratten**
   Obere Reihe (a, b): Renin-mRNA-Expression in Renin-transgenen Ratten
   Die Expression wurde unter Verwendung einer ³⁵S-markierten für menschliches Renin (a) bzw. für Maus-Renin (b) spezifischen Sonde bei aufeinanderfolgenden Schnitten desselben Gewebeblocks ermittelt. Die für Maus-Renin-mRNA spezifische Sonde kreuzhybridisiert zu nahezu 100 % mit Ratten-Renin-mRNA und weist daher endogene Ratten-Renin-Transkripte nach. Die für menschliches Renin spezifische Sonde kreuzhybridisiert zu lediglich 60 % mit Ratten-Renin-mRNA. Die Verwendung beider Sonden zeigt ausschließlich Renin-Transkripte im Bereich der glomerulären afferenten Arteriole (Pfeile). Dieses Ergebnis deutet auf eine gewebespezifische Expression beider unter der Kontrolle ihrer natürlichen Promotoren stehenden Gene in Renin-produzierenden Zellen der Niere hin.
   mittlere Reihe (c, d): Immunhistochemie des Renin-Proteins in der Niere einer Renin-transgenen Ratte
   Die immunhistochemischen Daten wurden unter Verwendung spezifischer Antikörper für menschliches Renin (c) (Galen et al., J. Biol. Chem. 254 (1979), 4848) bzw. für Ratten-Renin bei aufeinanderfolgenden Schnitten desselben Gewebeblocks ermittelt. Beide Antikörper erkennen ihr Substrat lediglich in granulären extraglomerulären Zellen (Pfeile), obwohl die Antikörper nicht völlig artspezifisch reagieren.
   Die mikroskopische Vergrößerung der Abbildungen a bis d beträgt 440 x.
   untere Reihe
   (e): Angiotensinogen-mRNA-Expression in der Leber einer Angiotensinogen-transgenen Ratte
   Das Ergebnis wurde unter Verwendung einer radioaktiv markierten, für menschliches Angiotensinogen spezifischen Sonde (vgl. Beispiel 2) ermittelt (0,05 mg antisense-RNA pro Gewebeschnitt, Expositionsdauer 8 Tage). Eine hohe Dichte der Silberkörnung wurde ausschließlich in Parenchymzellen nachgewiesen.
   L: Lumen der Hauptvene.
   (f): Angiotensinogen-mRNA-Expression in der Leber einer nicht-transgenen Sprague Dawley-Ratte
   Zum Nachweis der Angiotensinogen-mRNA wurde eine für Ratten-Angiotensinogen spezifische Sonde verwendet. Die Versuchsbedingungen waren ansonsten die gleichen wie für (e). Die Färbung des Gewebeschnitts ist deutlich schwächer als in (e). Zusätzliche Kontrollexperimente haben gezeigt, daß beide Sonden eine ähnliche Signalintensität erzeugen, wenn sie mit Angiotensinogen-Transkripten von Ratte und Mensch hybridisiert werden.
   Die mikroskopische Vergrößerung der Abbildungen e und f beträgt 660 x.

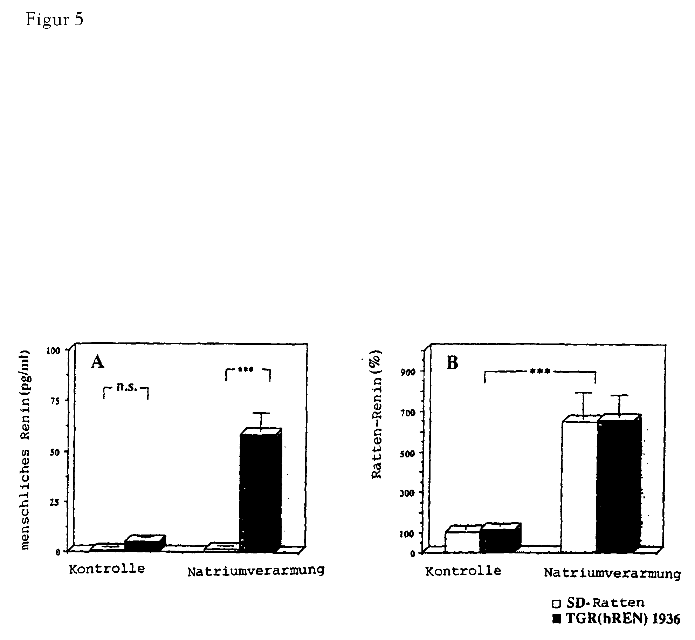

**Fig. 5: Reaktion von humanem und Rattenrenin auf Stimulation des RAS durch Natriumverarmung bei Renin-transgenen Ratten**
   Diagramm a) zeigt, daß die Menge an menschlichem Renin im Plasma nach Natriumverarmung nach Gabe von Furosemid signifikant ansteigt. Der Wert für menschliches Renin im Plasma der transgenen Ratten vor Natriumverarmung beträgt 4,9 pg/ml. Er liegt damit im selben Bereich wie im menschlichen Plasma. Nach Natriumverarmung durch Gabe von Furosemid steigt der Wert für menschliches Renin in TGR (hREN) 1936 transgenen Ratten auf über 50 pg/ml an. Als Kontrollen wurden nicht-transgene Ratten verwendet; es erfolgt bei ihnen keine Stimulation der Expression von menschlichem Renin.
   Diagramm b) zeigt, daß die Werte für endogenes Renin transgener als auch nicht-transgener Ratten um etwa einen Faktor 6 nach Natriumverarmung im Plasma ansteigen. Der Vergleich der Mengen an endogenem und transgenen Renin im Plasma transgener und nicht-transgener Ratten zeigt, daß die Werte weder vor noch nach Natriumverarmung signifikant verschieden und damit nicht abhängig von der Anwesenheit des Transgens sind.

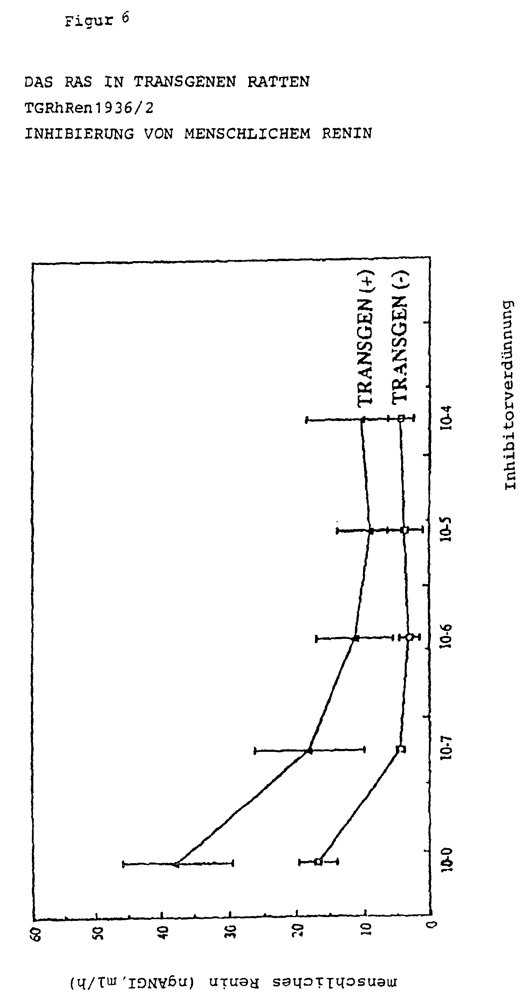

**Fig. 6: Spezifische Hemmung von transgenem Renin im Plasma von Renin-transgenen Ratten durch einen für menschliches Renin spezifischen Inhibitor**
   Der für menschliches Renin spezifische Inhibitor RO425892 wurde in Konzentrationen von 10⁻⁸ M bis 10⁻⁴ M mit Plasma von TGR(hREN) transgenen Ratten inkubiert. Das Diagramm zeigt, daß menschliches Renin bei einer Inhibitor-Konzentration von 10⁻⁶ vollständig gehemmt wird. Der Inhibitor reagiert nicht mit Ratten-Renin.

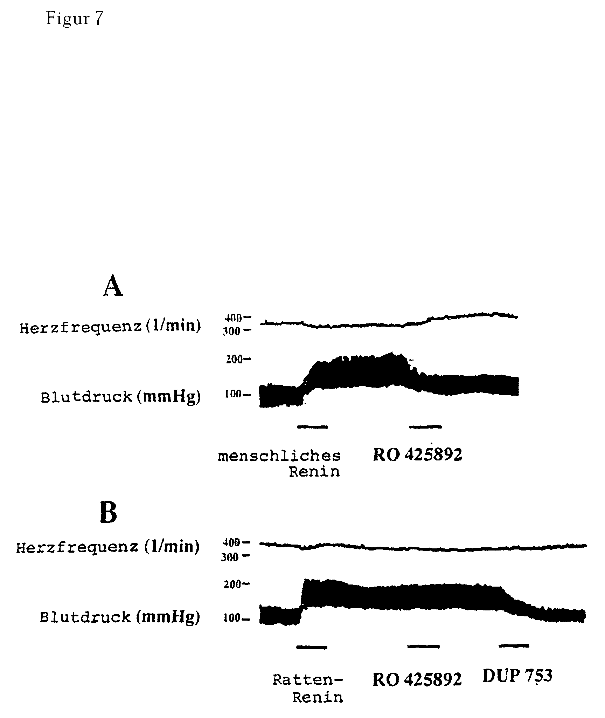

**Fig. 7: Auswirkung der Gabe von menschlichem Renin, Ratten-Renin, von RO425892 und von DUP 753 auf den Blutdruck Angiotensinogen-transgener Ratten**
   Darstellung des Blutdrucks und der Herzfrequenz in einer wachen freilaufenden Angiotensinogen-transgenen Ratte.
   A: Der Blutdruck steigt auf Plateauwerte von 200 mm Hg systolisch nach Infusion von gereinigtem menschlichem Renin, das in einer Dosierung von 5 µg ANG I/ml/h als Bolus über einen Zeitraum von 5 Minuten intravenös verabreicht wird. Die intravenöse Verabreichung von RO425892 in einer Konzentration von 1000 µg/kg Körpergewicht führt den Blutdruck innerhalb kurzer Zeit auf den Normalwert vor der Behandlung zurück.
   B: Die Infusion von Renin aus Rattennieren führt in einer Angiotensinogen-transgenen Ratte ebenfalls zu einem schnellen Anstieg des Blutdrucks auf einen Wert von etwa 200 mm Hg systolisch. Die Injektion von RO425892 in einer Konzentration von 1000 µg/kg Körpergewicht führt nicht zu einem Rückgang des Blutdrucks. Die intravenöse Verabreichung von 10 mg des Angiotensinrezeptor-Antagonisten DUP753 pro kg Körpergewicht als Bolus über einen Zeitraum von 5 Minuten führt zum Rückgang des Blutdrucks auf den Normalwert vor der Behandlung.
   Der Versuch wurde 48 Stunden nach Implantation von chronischen Kathetern in die Femoralarterie und Vene durchgeführt. Die Implantation des Katheters führte nicht zu einer Erhöhung des Blutdrucks. Der Blutdruck wurde mit einem Senso Nor 840 Wandler, Horten Electronics, Norwegen gemessen, der an einen Hellige Polygraph Recomed 330 P angeschlossen war.
   Die Herzfrequenz sinkt gegenregulatorisch zum Blutdruckanstieg um etwa 20-50 Schläge pro Minute ab, die arterielle Hypertonie ist daher nicht Folge eines herzfrequenzabhängigen Anstiegs des Herzzeitvolumens.

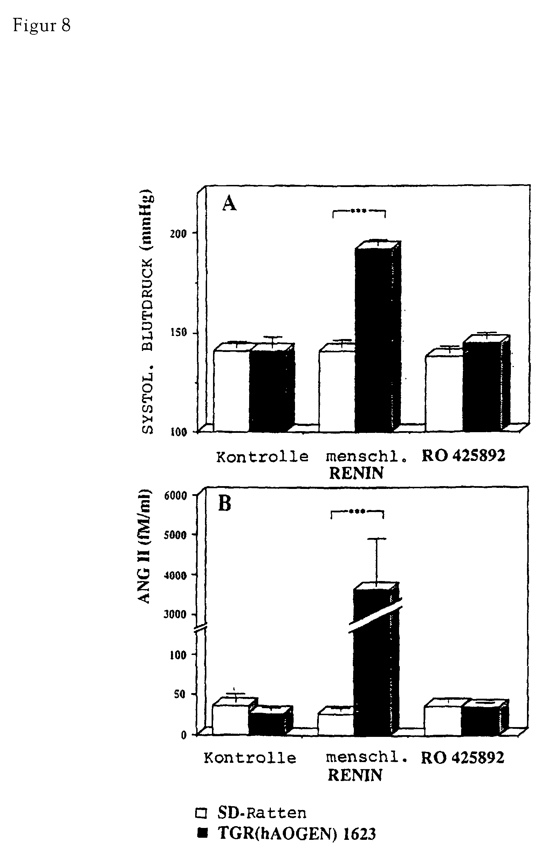

**Fig. 8: Auswirkung der Gabe von menschlichem Renin und von RO425892 auf den Blutdruck Angiotensinogen transgener Ratten**
   A: "Steady state" - Blutdruckwerte in Angiotensinogen-transgenen Ratten (dunkle Säulen) und nicht transgenen Geschwistern (helle Säulen) vor und 10 Minuten nach Bolus-Injektion von gereinigtem menschlichen Renin und für menschliches Renin spezifischem Inhibitor RO 425892. Als Reaktion auf die Verabreichung von menschlichem Renin sind die Blutdruckwerte bei den transgenen Ratten, nicht jedoch bei den nicht-transgenen Ratten deutlich erhöht. Die Gabe von RO 425892 führt bei den transgenen Ratten zum Rückgang des Blutdrucks auf Normalwerte, hat jedoch bei nicht-transgenen Ratten keine Auswirkungen auf den Blutdruck.
   B: Bestimmung der ANG II-Plasmawerte in Angiotensinogen-transgenen Ratten.
      Die Bestimmung der Plasmawerte für ANG II erfolgte während des unter A, oben, beschriebenen Experiments. Die Erzeugung von ANG II verläuft parallel zum Blutdruckanstieg. Die Infusion von menschlichem Renin ändert die ANG II-Werte in nicht-transgenen Ratten nicht. Dieses Ergebnis belegt die Artspezifität der in diesem Versuch getesteten RAS-Komponenten. Die Bildung von ANG I ging im wesentlichen mit der Bildung von ANG II einher.

Die Werte sind als Mittelwerte ± SEM (Standardfehler der Mittelwerte, "Standard Error of the Mean") mit n = 6 für jede Gruppe dargestellt. Die statistische Analyse wurde mittels ANOVA durchgeführt. *** bedeutet P<0,001. Die Dosierungen der für die Infusionen verwendeten Substanzen sind in der Legende zu Fig. 7 angegeben.

Die Verfahren zur Bestimmung der ANG II-Werte sind beschrieben in Schelling et al., Neuroendocrinology 31 (1980), 297-308 und in Hermann et al., Clin. Chem. 34/6 (1988), 1046-1051.

Mit den erfindungsgemäßen transgenen Ratten werden der Blutdruckforschung neue, bisher nicht verfügbare Möglichkeiten an die Hand gegeben. Versuche mit transgenen Labortieren wurden bisher überwiegend mit Mäusen durchgeführt. Ein Grund hierfür war, daß bei der Erzeugung transgener Ratten erhebliche technische Schwierigkeiten bestehen, die mit der Superovulation und der Vitalität der Eier zusammenhängen. Ratten bieten jedoch für die Herz-Kreislauf-Forschung gegenüber Mäusen wesentliche Vorteile: Zum einen eignen sie sich aufgrund der Verfügbarkeit von Techniken zur Blutdruckmessung, die bei der Maus nicht anwendbar sind, wesentlich besser für analytische und funktionelle Untersuchungen. Darüber hinaus ist es bei Mäusen praktisch unmöglich, chronische Verlaufsbeobachtungen der Blutdruckentwicklung durchzuführen. Akute Experimente mit invasiven Methoden sind mit großen instrumentellen und methodischen Schwierigkeiten behaftet und führen nach dem Experiment im allgemeinen zum Tod der Tiere. Endokrinologische Untersuchungen sind unter anderem wegen der Schwierigkeit, ausreichende Mengen Blut zu gewinnen, nur in sehr beschränkter Weise möglich. Für Ratten stehen des weiteren die mit Abstand größten vergleichenden pathophysiologischen und pharmakologischen Daten im Zusammenhang mit dem Herz-Kreislauf-System zur Verfügung. Noch wichtiger ist allerdings die Tatsache, daß Rattenmodelle sowohl für die sekundär als auch für die primär genetisch bedingte Form der Hypertonie existieren, von denen die bekanntesten die Stämme der spontan-hypertensiven SHR-Ratten sind. Die Methodik zur Untersuchung akuter und chronischer Veränderungen der Herz-Kreislauffunktion bei Ratten, endokrinologische, pharmakologische, pathophysiologische und elektro-physiologische Untersuchungsmethoden sind hervorragend ausgearbeitet und können eingesetzt werden. Aus diesem Grund eignen sich Ratten insbesonders auch für die Entwicklung neuer Pharmaka für Herz-Kreislauferkrankungen. Zudem wird Bluthochdruck in Ratten aus konventionellen Gründen in gleicher Weise definiert wie beim Menschen. Es bestehen darüber hinaus keine Unterschiede hinsichtlich der Blutdruckwerte zwischen Mensch und Ratte.

Mit den erfindungsgemäßen transgenen Ratten ist es erstmals möglich, Veränderungen des Blutdrucks mit der Wirkung von bestimmten Expressionsprodukten von Genen, deren Genprodukte an der Blutdruckregulation beteiligt sind, direkt in Beziehung zu setzen. Überraschenderweise konnte nämlich erfindungsgemäß gezeigt werden, daß menschliches Renin und menschliches Angiotensinogen nicht mit Ratten-Renin und Ratten-Angiotensinogen interagieren. Da im Stand der Technik bekannt war, daß Ratten-RAS Genprodukte mit Maus-RAS-Genprodukten kreuzreagieren (Mullins et al. a.a.O., Ohkubo et al. a.a.O.) war zu erwarten, daß auch die menschlichen RAS-Genprodukte mit dem Ratten-RAS-Genprodukten interagieren. Die erfindungsgemäßen transgenen Ratten weisen somit einen deutlichen unerwarteten Vorteil gegenüber den im Stand der Technik bekannten transgenen Ratten und Mäusen, die RAS-spezifische Gene in ihrem Genom tragen, sowie gegenüber den spontan hypertensiven Ratten (SHR) mit genetischem Hochdruck auf. Während der Bluthochdruck im SHR-System auf die Interaktion von Genprodukten von schätzungsweise 4 bis 8 Ratten-Genen zurückzuführen ist und sich dadurch allenfalls analoge Schlüsse zum Bluthochdruck beim Menschen ziehen lassen, beruht der erhöhte Blutdruck bei den erfindungsgemäßen transgenen Ratten auf der Interaktion nur zweier (menschlicher) Genprodukte. Das erfindungsgemäße transgene System wird daher in seiner Aussagekraft nicht durch die Einflüsse endogener Genprodukte gestört.

Die erfindungsgemäßen transgenen Ratten tragen zwei menschliche Gene in ihrem Genom, dessen Genprodukte an der Blutdruckregulation beteiligt sind. Die erfindungsgemäßen transgenen Ratten, die zwei menschliche Gene in ihrem Genom, tragen können für beide Gene oder nur für ein Gen homo- oder heterozygot sein.

Die transgene Ratte weist in etwa ebenso hohe und vorzugsweise erhöhte Werte der transgenen Produkte im Plasma im Vergleich zu den Werten im menschlichen Plasma auf. Die Expression beider Transgene erfolgt gewebespezifisch. Die Aktivität des transgenen Renins ist durch einen für menschliches Renin spezifischen Inhibitor hemmbar.

Durch die Anwesenheit von beiden menschlichen Transgenen im Genom einer Ratte wird der Blutdruck z. T. abhängig vom menschlichen transgenen RAS. Die transgenen Genprodukte sind prinzipiell körpereigene Produkte. Aufgrund ihrer Anlage bereits in der Eizelle treten immunologische Nebenwirkungen nicht auf.

Mit der Verwendung der erfindungsgemäßen transgenen Ratten für die Testung z.B. pharmakologischer blutdrucksenkender Substanzen werden bis dato durchgeführte Tierversuche an höheren Tieren, z.B. Affen, aber auch an Freiwilligen (Menschen), wesentlich eingeschränkt werden können bzw. unnötig gemacht. Gleichzeitig werden so die Entwicklungskosten für neue Medikamente im Blutdrucksektor deutlich gesenkt.

Die erfindungsgemäßen transgenen Testsysteme erlauben darüber hinaus gegenüber in der Praxis gängigen in vitro Vorversuchen eine schnellere und aussagekräftigere Entwicklung von Blutdruckmitteln und besitzen damit einen hohen praktischen Wert für die Entwicklung besserer und spezifischerer Pharmaka. Beispielsweise kann davon ausgegangen werden, daß die mit den erfindungsgemäßen transgenen Ratten mögliche Verbesserung der Entwicklung von Renin-Inhibitoren ihren Niederschlag in optimierten Therapiemöglichkeiten des Bluthochdrucks findet.

Des weiteren erlauben die erfindungsgemäßen transgenen Ratten neuartige Ansätze in der Aufklärung der pathophysiologischen Ursachen des Bluthochdrucks im Tiermodell. Am Tiermodell können somit humanspezifische Medikamente zur kausalen Blutdrucktherapie entwickelt werden.

Die Beispiele erläutern die Erfindung.

### Beispiel 1

### Erzeugung transgener Ratten, die das menschliche Renin-Gen in ihrem Genom tragen.

Ein 17,6 kb DNA-Fragment, das das gesamte menschliche chromosomale Renin-Gen (10 Exons und 9 Introns, Miyazaki et al. a.a.O.) einschließlich 3 kb 5'-flankierender Sequenz mit möglichen regulatorischen Sequenzen inklusive des Promotors und 1,2 kb 3'-flankierender Sequenz wurde in einen modifizierten pUC19-Plasmidvektor cloniert (Fig. 1), wie in Fukamizu et al., a.a.O., beschrieben.

Nach Vermehrung der rekombinanten DNA und anschließender Reinigung ( Sambrook et al., Molecular Cloning, 2. Ausgabe 1989, Cold Spring Harbor Laboratory, Cold Spring Harbor) wurde das Renin-Gen durch Spaltung mit Restriktionsendonucleasen (BglII, ClaI) von der Vektor-DNA abgetrennt. Das linearisierte Renin-Gen wurde nach dem Verfahren von Gordon et al., Proc. Natl. Acad. Sci. USA 77 (1980), 7380 - 7384, in männliche Vorkerne befruchteter Eizellen injiziert. Dazu wurden Sprague Dawley (Auszucht)-Ratten mit Wistar Kyoto (Inzucht)-Ratten gepaart und die befruchteten Eizellen für die Injektionen entnommen.

Es wurden 87 injizierte Eizellen nach dem von Hogan et al., in "Manipulating the mouse embryo", (1986) (Herausgeber: Hogan),The Cold Spring Harbor Laboratory, Cold Spring Harbor, beschriebenen Verfahren in weibliche pseudoschwangere Sprague Dawley-Ratten (eigene Zucht) reimplantiert. Es wurden 15 Nachkommen ausgetragen, die auf Präsenz des Transgens untersucht wurden. Dazu wurden den neugeborenen Ratten durch eine Biopsie Gewebeproben aus dem Schwanz entnommen und DNA daraus nach Standardverfahren isoliert. Die DNA wurde mit dem Restriktionsenzym PvuII partiell gespalten und auf einem 0,8 % Agarosegel elektrophoretisch aufgetrennt. Nach dem Transfer der DNA auf einen Membranfilter (E. Southern, J. Mol. Biol. 98 (1975) 503) wurde die DNA mit einer für menschliches Renin spezifischen Sonde, einem nach dem Verfahren von Feinberg und Vogelstein (Sambrook et al., a.a.O.) radioaktiv markierten 300 Basenpaaren (bp) langen Fragment aus dem cDNA-Clon pDDID2 (Field et al., Hypertension 6 (1984), 597) unter stringenten Bedingungen hybridisiert, anschließend unter stringenten Bedingungen gewaschen und autoradiographiert. Bei DNAs von 2 der Nachkommen wurden Hybridisierungssignale der erwarteten Länge gefunden, die für das menschliche Renin spezifisch sind. Dies bedeutet, daß zwei der Nachkommen das menschliche Renin-Transgen im Genom tragen. Diese Tiere wurden mit TGR (hREN) 1936 und TGR (hREN) 1988 bezeichnet. Beide Tiere vererbten das Transgen auf die Nachkommen. In der von TGR (hREN) 1936 abstammenden Linie liegt das Transgen in der F3-Generation im homozygoten Zustand vor.

### Beispiel 2

### Erzeugung transgener Ratten, die das menschliche Angiotensinogen-Gen in ihrem Genom tragen

Aus menschlichem Plazenta-Gewebe wurde nach dem bei Blin und Stafford, Nucleic Acids Res. 3 (1976), 2303 beschriebenen Verfahren genomische DNA isoliert. Nach Standardverfahren (Sambrook et al., a.a.O.) wurde die DNA nach partieller Spaltung mit dem Restriktionsenzym Sau 3A im Phagen Charon 28 cloniert. Etwa 6 x 10⁵ rekombinante Phagen dieser Genbank wurden ausplattiert und mit einem 1262 bp langen BstEII-Fragment, das aus Phag3 (Kageyama et al., Biochemistry 23 (1984) 3603-3609) isoliert wurde, als Sonde nach dem Angiotensinogen-Gen abgesucht.

Dazu wurden die rekombinanten Phagen-DNAs nach Standardverfahren (Sambrook et al., a.a.O.) auf Membranfilter übertragen und mit der Sonde 16 Stunden bei 65°C hybridisiert.

Als Puffer wurde eine 5x SSPE-Lösung (1xSSPE = 0,15 M NaCl, 0,01 M NaH₂PO₄·H₂O, 1 mM EDTA, pH 8,4), 1x Denhardt'sche-Lösung (0,02 % BSA, 0,02 % Polyvinylpyrrolidon, 0,02 % Ficoll), 0,1 % SDS, 100 µg/ml denaturierte Lachs-Sperma-DNA, 5 x 10⁶ cpm/ml radioaktiv markierte Sonde verwendet. Nach der Hybridisierung wurden die Filter zweimal in 2 x SSC (1XSSC = 0,15 M NaCl, 0,015 M Natriumcitrat, pH 7,0) bei Raumtemperatur und zweimal in 2 x SSC und 0,1 % SDS 30 Min. bei 65°C gewaschen. Die Filter wurden getrocknet und bei -70°C autoradiographiert.

Aus dem rekombinanten Phagen IhAG-1, der ein Hybridisierungssignal mit der Sonde zeigte, wurden ein 3,5 kb EcoRI/BamHI-Fragment, ein 5,0 kb BamHI-Fragment und ein 0,5 kb BglII-Fragment in geeignete Spaltstellen in der Polylinkerregion des Plasmidvektors pUC19 subcloniert. Die entsprechenden rekombinanten Plasmide erhielten die Bezeichnungen phAG35EB, phAG50b und phAG85G. Aus phAG85G wurde ein genomisches 2,7 kb BamHI-Fragment in die BamHI-Spaltstelle von pUC19 cloniert. Das rekombinante Plasmid wurde mit phAG272B bezeichnet.

Die Struktur des menschlichen Angiotensinogen-Gens wurde aufgeklärt:
Exon-tragende DNA-Fragmente und die 5'-flankierenden und 3'flankierenden Bereiche wurden durch Restriktionskartierung identifiziert. Nach Subclonierung in M13 oder pUC19- wurde die Primärstruktur der Exons, der Exon/Intron-Übergänge und der flankierenden Bereiche durch das Ketten-Abbruch-Verfahren (Sanger et al., Proc. Natl. Acad. Sci. USA 74 (1977) S. 5463) sequenziert.

Die Nucleotidsequenz aller Exon-Intron-Übergänge wurde bestimmt. Das menschliche Angiotensinogen-Gen besteht aus 5 Exons und 4 Introns, was durch Vergleiche mit der bekannten cDNA-Sequenz (Kageyama et al., a.a.O.) gezeigt werden konnte.

Die Konstruktion des für die Erzeugung der transgenen Ratten verwendeten Plasmids pHAgTM14 ist in der Arbeit von Fukamizu et al., a.a.O. dargestellt. Die Restriktionskarte des Plasmids pHAgTM14 ist in Figur 2 dargestellt.
Das 16,3 kb großes DNA-Fragment, das das gesamte menschliche Angiotensinogen-Gen einschließlich 1,1 kb 5'-flankierender und 2,4 kb 3'-flankierender Sequenzen enthält, wurde nach Abspaltung der pUC19-Vektor-DNA nach dem in Beispiel 1 beschriebenen Verfahren zur Erzeugung transgener Ratten verwendet. Die Ratten-DNAs wurden, wie in Beispiel 1 beschrieben, mit einer radioaktiv markierten, für menschliches Angiotensinogen spezifischen Sonde auf Anwesenheit transgener Angiotensinogen-DNA untersucht.
4 Ratten, bezeichnet mit TGR(hAOGEN) 1623, 1663, 1670 und 1671, hatten das Transgen in ihr Genom inkorporiert und vererbten es auf die Nachkommenschaft. In den von TGR (hAOGEN) 1623 und 1670 abstammenden Linien liegt das Transgen in der F3-Generation in homozygotem Zustand vor.

### Beispiel 3

### Erzeugung transgener Ratten, die in ihrem Genom das menschliche Renin- und das menschliche Angiotensinogen-Gen tragen

Nachkommen der F1-Generation von TGR(hAOGEN) 1670 wurden mit Nachkommen der F1-Generation von TGR (hREN) 1936 gekreuzt. Von den 8 Nachkommen aus dieser Kreuzung trugen 4 keines der Transgene in ihrem Genom, einer trug das Renin-Transgen, einer das Angiotensinogen-Transgen und 2 beide Transgene.

### Beispiel 4

### Test auf gewebespezifische Expression der Transgene

Die Expression der transgenen mRNA in unterschiedlichen Geweben wurde mit einem "RNase-Protection-Assay" (Sambrook et al., a.a.O.) untersucht. Für den Nachweis der transgenen Renin-Expression wurde ein mit ³²P markiertes RNA-Transkript durch Transkription einer 291 Nucleotide langen antisense-RNA von einem im pGEM4-Vektor subclonierten SacI/EcoRV-Fragment, das aus einem menschlichen Renin-cDNA-Clon stammt, unter Verwendung von T7-RNA-Polymerase erzeugt. Dieses Transkript enthält 225 Nucleotide menschlicher Renin-antisense-RNA und 66 Nucleotide der vom Vektor codierten Sequenz.

Für den Nachweis der transgenen Angiotensinogen-Expression wurde von einem im Vektor pGEM5 subclonierten StuI/AvaII-Fragment der menschlichen Angiotensinogen-cDNA transkribierte antisense-RNA verwendet. Zur Transkription wurde T7-RNA-Polymerase verwendet. Das Transkript umfaßte 361 Nucleotide der antisense-RNA und 51 Nucleotide der vom Vektor codierten Sequenz.

Für die Hybridisierung mit den für menschliches Renin bzw. Angiotensinogen spezifischen antisense-RNAs wurde Gesamt-RNA nach dem in Auffray und Rougeon, Eur. J. Biochem. 107 (1980) 303-314 beschriebenen Verfahren durch Fällung mit Lithiumchlorid isoliert. Proben getrockneter RNA wurden in 30 µl 80 % Formamid, enthaltend 40 mM PIPES (pH 6,8), 400 mM NaCl, 1 mM EDTA und 2 x 10⁵ cpm des gelgereinigten antisense-Transkripts gelöst. Nach Hitzedenaturierung (60 sek. bei 95°C) und 20 Stunden Inkubation bei 50°C wurde der RNase Verdau in 300 µl Puffer enthaltend 40 µg/ml RNase A (Sigma) und 2 µg/ml RNase T1 (Calbiochem) 45 Minuten bei 37°C durchgeführt.

Die gegen RNase-Abbau geschützten RNA-Sequenzen (Hybride aus RNA und radioaktiv markierter Sonde) wurden nach Verdau der Enzyme durch Proteinase K durch Gelelektrophorese auf einem 8 M Harnstoff/5 % Polyacrylamid-Gel analysiert. Nach der Elektrophorese wurden die Gele getrocknet und bei -70°C autoradiographiert.

Die Ergebnisse dieser Untersuchungen sind in Figur 3 dargestellt und in den Tabellen I und II zusammengefaßt. Sie zeigen, daß transgene Renin-mRNA vorzugsweise in der Niere, in der Lunge und im Darm exprimiert wird. Im Herz und in der Leber konnte dagegen keine Expression gezeigt werden. Transgene Angiotensinogen-mRNA wurde vorzugsweise in der Leber, Niere, im Gehirn, im Herz und Jejunum nachgewiesen.

Das durch einen Northern Blot (Sambrook et al., a.a.O.) und anschließende Hybridisierung mit einem 298 bp ApaI/EcoRI-Fragment aus dem Plasmid pHag3 (Kageyama et al., a.a.O.) in den Nieren nachgewiesene Transkript des transgenen Renin-Gens entsprach in seiner Größe von 16S dem in menschlichen Nieren gefundenen. Dieses Transkript konnte nicht in nicht-transgenen Kontroll-Ratten detektiert werden.

Die Expression des transgenen Renins in der Niere und des transgenen Angiotensinogens in der Leber wurde durch immunhistochemische Experimente bzw. durch in situ-Hybridisierungsexperimente bestätigt (Figur 4). Darüber hinaus wurde mit in situ-Hybridisierungsexperimenten Angiotensinogen-mRNA-Expression in der Niere und im Gehirn nachgewiesen.

Für die in situ-Hybridisierungen wurden den Tieren eine Kanüle in die Aorta eingeführt. Die Ratten wurden anschließend durch Perfusion mit 3 % Paraformaldehyd in PBS 5 Minuten lang fixiert.
Für die immunhistochemischen Experimente wurden die Nieren 12 Stunden lang nachfixiert und in Paraffin eingebettet.
Auf die durch eine Xylol/Alkoholreihe deparaffinierten Schnitte wurden spezifische polyclonale Kaninchen-Antikörper in Verdünnungen von 1:1000 bis 1:10 000 aufgetragen. Gebundener Antikörper wurde mit einem Peroxidase-Antiperoxidase-System unter Verwendung von Diaminobenzidin und 0,02 % H₂O₂ nachgewiesen (Sternberger et al., Immunochemistry (1979), John Wiley & Sons, New York). Die Gewebe wurden mit Hämatoxylineosin gegengefärbt.
Für die in situ Hybridisierungen wurden die Organe in Sucrose-PBS (800 mosmol) gespült und Schock-gefroren. Um die Renin-mRNA-Expression nachzuweisen, wurde ein 330 bp SacI/PstI-Fragment aus der Maus-Renin-cDNA in den Vektor pSP65 subcloniert. Das rekombinante Plasmid wurde durch Spaltung mit AccI linearisiert und antisense-RNA durch Transkription mit T7-RNA-Polymerase hergestellt. Für den Nachweis der Angiotensinogen-mRNA wurde ein 290 bp langes PvuII/BamHI-Fragment aus Ratten-Angiotensinogen-cDNA in den Vektor pGEM4 subcloniert, das rekombinante Plasmid wurde durch Spaltung mit EcoRI linearisiert und antisense-RNA durch Verwendung von Sp6-Polymerase hergestellt. Die in situ-Hybridisierungen wurden nach dem in Bachmann et al., Histochemistry 94 (1990), 517-523 beschriebenen Verfahren durchgeführt. Die höchste Stringenz beim Waschen war 0,1 x SSC bei 48°C (20 Minuten). Die Gewebeschnitte wurden mit Hämatoxylineosin gegengefärbt.

Die Ergebnisse sind in den Tabellen I (Renin) und II (Angiotensinogen) zusammengefaßt.

Das Expressionsmuster für transgenes Renin und transgenes Angiotensinogen in Ratten, die sowohl das Renin- als auch das Angiotensinogen-Gen tragen, entspricht für jedes Transgen dem des in nur ein Transgen tragenden Ratten gefundenen.

### Beispiel 5

### Bestimmung der Plasmawerte für transgenes Renin und transgenes Angiotensinogen

Die Bestimmung der Werte für menschliches Renin im Plasma Renin-transgener Ratten erfolgte durch einen direkten immunoradiometrischen Test (IRMA). Darin wurden 2 Paare monoclonaler Antikörper verwendet, wobei ein Paar spezifisch für aktives menschliches Renin (3E8 und 4G1 [Menard et al., J. Hypertens. Suppl. 3 (1985), 275-278]) und das andere Paar spezifisch für Gesamt-Renin ist (3E8 und 4E1 [Toffelmire et al., J. Clin. Invest. 83 (1989), 679-687]).

250 µl Ratten-Plasma wurde mit etwa 20 mg Magnogel (Pasteur, Frankreich), an das monoclonaler Antikörper 3E8 nach Standardverfahren gekoppelt worden war gemischt. 3E8 erkennt sowohl aktives Renin als auch Prorenin, die zusammen als Gesamt-Renin bezeichnet werden. Die Proben wurden 2 Stunden bei Raumtemperatur auf einem Magnelio-Schüttler (Pasteur, Frankreich) inkubiert. Anschließend wurden 2 ml Waschpuffer (0,002 M Imidazol enthaltend 0,1 % Pferdeserum, 0,0001 % NaN₃ und 0,0001 % Farbe) 2 Minuten lang zugegeben. Das Magnogel wurde mittels eines Magneten fixiert, der Überstand wurde verworfen. Nach einem zweiten Waschschritt wurden 250 µl (250 000 cpm) mit ¹²⁵J markiertem monoclonalen Antikörper 4G1 für den Nachweis von aktivem menschlichen Renin (der Antikörper bindet am aktiven Zentrum des Enzyms) oder 250 µl (250 000 cpm) mit ¹²⁵J markiertem monoclonalen Antikörper 4E1 für den Nachweis von Gesamt-Renin zugegeben. Nach 3-stündiger Inkubation bei Raumtemperatur wurden die Proben 3 mal wie oben beschrieben gewaschen. Die Proben wurden 2 Minuten in einem Gamma-Zähler (Berthold, BRD) ausgewertet. Die Bestimmung der Ratten-Renin-Konzentration erfolgte wie in Schelling et al., a.a.O. und Hermann et al., a.a.O. beschrieben.

Die Ergebnisse sind in Tabelle I zusammengefaßt.

In Renin-transgenen Ratten, nicht aber in nicht-transgenen Kontroll-Ratten wurden hohe Plasma-Werte für menschliches Renin nachgewiesen. In der Ratte TGR (hREN) 1988 betrug der Wert für aktives Renin 226 pg/ml, in der Ratte TGR (hREN) 1936 betrug er 4,9 pg/ml. Die durchschnittliche humane Renin-Aktivität im Ratten-Plasma beträgt 18 ng ANG I/ml/h. Der durchschnittliche Wert im menschlichen Plasma beträgt 25,1 ng ANG I/ml/h, bewegt sich also in der gleichen Größenordnung. Die Werte für Ratten-Prorenin und Ratten-Renin waren im Vergleich zu nicht transgenen Kontrollratten unverändert.

Die Werte für Angiotensinogen, Angiotensin I und Angiotensin II sind in Renin-transgenen Ratten im Vergleich zu nicht-transgenen Kontroll-Ratten nicht signifikant verändert. Dies belegt, daß Ratten-Angiotensinogen nicht von menschlichem Renin gespalten wird.

Die Bestimmung der Angiotensinogen-Werte im Plasma erfolgte mit einem ELISA. Dazu wurden die Vertiefungen von "Immunoplates" (STL, Overath, BRD) 16 Stunden bei 4°C mit 100 µl des für menschliches Angiotensinogen spezifischen monoclonalen Antikörpers H₁₀F₁₀ (0,5 µg/ml) inkubiert.

Die weiteren Schritte wurden bei Raumtemperatur durchgeführt. Nach 3 Waschschritten mit PBS (phosphatgepufferte Kochsalzlösung) enthaltend 0,5 % Tween 20® wurden die freien Bindungsstellen auf den Platten 1 Stunde mit 200 µl BSA-Lösung (im oben beschriebenen PBS-Tween-Puffer) blockiert. Die Platten wurden anschließend, wie oben beschrieben, 3 mal gewaschen. Anschließend wurden die Vertiefungen mit je 100 µl der Plasmaproben versetzt und 2 Stunden inkubiert.

Nach 3 weiteren Waschschritten wurden die Vertiefungen mit 100 µl einer 1:2000 Verdünnung in PBS-Tween des für menschliches Angiotensinogen spezifischen polyclonalen Kaninchen-Antikörpers 4H69.6 (Hilgenfeldt et al., Eur. J. Clin. Pharmacol. 38 (1990), 125-131) 2 Stunden inkubiert. Die Platten wurden anschließend extensiv gewaschen und die Vertiefungen mit Peroxidase-gekoppeltem Ziegen-anti-Kaninchen IgG (Sigma, München), der nach Anleitung des Herstellers gebrauchsfertig gemacht worden war, versetzt und 2 Stunden inkubiert.

Nach weiteren 5 Waschschritten wurde die Farbreaktion durch Zugabe von einer o-Phenylendiaminlösung in Natriumphosphatpuffer (0,1 M, pH 7,5) enthaltend 6,5 M H₂O₂ gestartet. Die enzymatische Reaktion wurde nach 10 Minuten durch Zugabe von 100 µl 12 % H₂SO₄ gestoppt.

Die Farbreaktion wurde bei 492 nm mit einem "ELISA-Reader EAR 400" (SLT-Labinstruments, Österreich) gemessen. Rattenangiotensinogen wurde nach Inkubation von Plasma mit Schweinerenin (1 h bei 37°C) indirekt bestimmt. Die Menge an Angiotensin I wurde mittels eines ANG I-RIA gemessen (Hermann et al., a.a.O.). Die Gesamtmenge von ANG I entspricht der Menge an Rattenangiotensinogen. Die Ergebnisse sind für Tiere der Linien 1663 und 1670 in Tabelle II zusammengefaßt. Die Werte stellen den Mittelwert und die Standardabweichung bei 6 Tieren für jede Bestimmung dar. Die Plasmawerte für Angiotensinogen waren in allen 4 Angiotensinogen-transgenen Ratten erhöht und lagen im Bereich zwischen 125 µg/ml (1671) und 3000 µg/ml (1623). In nicht-transgenen Kontroll-Ratten wurde kein menschliches Angiotensinogen im Plasma gefunden. Die Plasmawerte für Ratten-Prorenin, -Renin, -Angiotensin I, -Angiotensin II und -CE wurden durch die Expression des Angiotensinogen-Transgens nicht beeinflußt. Dieses Ergebnis belegt, daß menschliches Angiotensinogen, selbst bei hohen Plasmakonzentrationen, kein Substrat für Ratten-Renin ist.

### Beispiel 6

### Einfluß von Natriumverarmung durch Gabe von Furosemid auf die Renin-Werte im Plasma

Natriumverarmung in den Ratten wurde durchgeführt durch eine intraperitoneale Injektion von 120 mg/kg Körpergewicht Furosemid und gleichzeitiger Gabe einer natriumarmen Diät von 10 mOsmol/Tag für sieben Tage. Die Blutproben wurden vor Beginn der Natriumverarmung und sieben Tage nach Behandlung durch retroorbitale Punktion unter leichter Etheranästhesie gewonnen.

Die Ergebnisse dieser Versuche sind in Figur 5 dargestellt. Sie zeigen, daß der transgene Renin-Spiegel im Plasma Renin-transgener Ratten nach Natriumverarmung um etwa einen Faktor 10 ansteigt. Ein Anstieg der endogenen Renin-Werte um einen Faktor 5 wurde nach Natriumverarmung sowohl in Renin-transgenen als auch in nicht-transgenen Ratten beobachtet. Der Anstieg der Werte für transgenes Renin blieb ohne Einfluß auf die endogenen Angiotensinogen-Werte. Dies ist ein weiterer Beleg für die Artspezifität der untersuchten RAS-Systeme. Tiere mit erhöhtem transgenen Renin-Spiegel können für einen empfindlichen Assay für die Reaktion und Testung von Hemmern des Renin-Angiotensin-Systems nach Infusion von menschlichem Angiotensinogen verwendet werden.

In einem weiteren Versuch wurde Plasma der Renin-transgenen Ratte TGR(hREN)1936 nach Natriumverarmung mit dem für menschliches Renin spezifischen Inhibitor RO425892 inkubiert. Diese Inkubation führte zu einer vollständigen Hemmung der transgenen Renin-Aktivität bei einer Inhibitor-Konzentration von 10⁻⁶ M, hatte jedoch keine Auswirkung auf die Aktivität des endogenen Renins (Figur 6). Diese Ergebnisse unterstützen die in vivo-Befunde und erlauben die in vivo - in vitro-Analoganalyse.

### Beispiel 7

### Bestimmung des Blutdrucks in Renin- und/oder Angiotensinogen-transgenen Ratten

Der Blutdruck wurde in Ratten auf zwei verschiedene Arten gemessen:
1. Schwanzplethysmographisch: Hierzu werden die Ratten durch Ether leicht narkotisiert. Der Rattenschwanz wird in einen plethysmographischen Schlauch eingeführt und mit einem Manometer der Druck in dem Schlauchsystem erhöht. Sobald der systolische Blutdruck höher ist als der plethysmographische Druck, kann dieses an einer Wassersäule abgelesen werden.
2. Für direkte arterielle Blutdruckmessungen wird die Arteria femoralis freigelegt. Ein Polyethylen-Schlauch wird in die Arteria femoralis eingeführt und eingebunden. Der Polyethylen-Schlauch wird mit einer anticoagulierenden Heparinlösung gefüllt und an einen Blutdruckwandler angeschlossen, der den Druck in elektrische Impulse umwandelt. Diese werden auf einem elektronischen Schreibgerät dann registriert. Eine genauere Beschreibung dieses Verfahrens findet sich in Rasches et al., "Hypertensive Mechanism", F.K. Schattauer Verlag, Stuttgart-New York, 1982, S. 779-797 und den darin beschriebenen Referenzen.

Die Messung des Blutdrucks in transgenen Ratten, die entweder nur das Renin-Transgen oder nur das Angiotensinogen-Transgen im Genom tragen, ergab, daß der Blutdruck im Normbereich von 90 mm Hg diastolisch und 140 mm Hg systolisch lag. Ratten, die beide Transgene in ihrem Genom trugen, wiesen zeitweise einen erhöhten Blutdruck im Bereich von 155 mm Hg systolisch auf.
Der Blutdruck wurde hierbei intraarteriell nach Plazierung eines Katheters in der Arteria femoralis am wachen, freilaufenden Tier gemessen (direkte arterielle Blutdruckmessung).

### Beispiel 8

### Einfluß des für menschliches Renin spezifischen Inhibitors RO425892 und des Angiotensin-Rezeptor-Antagonisten DUP753 auf den Blutdruck Renin-transgener Ratten

RO425892 wurde Renin-transgenen Ratten nach Natriumverarmung in einer Konzentration von 1000 µg/kg Körpergewicht verabreicht. Die Gabe des Inhibitors hatte keinen Effekt auf den Blutdruck. Auch dieses Ergebnis belegt, daß menschliches Renin nicht mit Ratten-Angiotensinogen reagiert.

In einem weiteren Versuch bekamen die Renin-transgenen Ratten nach Natriumverarmung eine Infusion mit dem Angiotensin-Rezeptor-Antagonisten DUP753 (10 mg/kg Körpergewicht). Als Folge dieser Infusion wurde der Blutdruck um ± 15 mm Hg gesenkt, da DUP 753 sowohl mit dem menschlichen als auch mit dem Ratten-RAS reagiert.

### Beispiel 9

### Einfluß der Gabe von menschlichem Renin auf den Blutdruck bei Angiotensinogen-transgenen Ratten

Den Angiotensinogen-transgenen Ratten 1623 und 1670 wurde gereinigtes menschliches Nieren-Renin und rekombinantes humanes Renin intravenös bei einer Konzentration von 5 µg ANG I/ml/h verabreicht. Die Renin-Gabe führte dosisabhängig zu einer Erhöhung des systolischen Blutdrucks (Figuren 7 und 8).

Diese Blutdruckerhöhung konnte durch orale oder intravenöse Vorbehandlung der Angiotensinogen-transgenen Ratten mit dem für menschliches Renin spezifischen Inhibitor RO425892, der in einer Menge von 1,5 mg/kg Körpergewicht verabreicht wurde, verhindert werden.

Die Gabe von Ratten-Renin führte in diesen Ratten ebenfalls zu erhöhten Blutdruckwerten, diese Erhöhung konnte jedoch nicht durch Gabe des für menschliches Renin spezifischen Inhibitors RO425892 verhindert werden. Die Blutdruckerhöhung nach Gabe von Ratten-Renin konnte allerdings durch Gabe der das konvertierende Enzym inhibierenden Substanz Captopril (wirkt nicht artspezifisch) und den Angiotensin II-Rezeptor-Antagonisten DUP753 (wirkt ebenfalls nicht artspezifisch) rückgängig gemacht werden.

In nicht-transgenen Kontrollratten konnte lediglich eine Blutdruckerhöhung nach Gabe von Ratten-Renin, nicht aber nach Gabe von menschlichem Renin festgestellt werden.

Die Infusion von Ratten-Renin erhöht den Blutdruck in nicht-transgenen Ratten durch artspezifische Interaktion mit endogenem Angiotensinogen. Menschliches Renin reagiert dagegen nicht mit Ratten-Angiotensinogen und führt daher nicht zu erhöhtem Blutdruck.

## Patentansprüche

1. Transgene Ratte, **dadurch gekennzeichnet, daß** ihr Genom die beiden exprimierbaren menschlichen Gene Renin und Angiotensinogen enthält.

2. Transgene Ratte nach Anspruch 1, **dadurch gekennzeichnet, daß** mindestens eines der Gene unter der Kontrolle eines homologen Promotors steht.

3. Transgene Ratte nach Anspruch 1, **dadurch gekennzeichnet, daß** mindestens eines der Gene unter der Kontrolle eines heterologen Promotors steht.

4. Transgene Ratte nach einem der Ansprüche 1 bis 3, die einen durch die eingeführten Gene verursachten erhöhten Blutdruck, vorzugsweise von größer als 90/140 mm Hg, oder einen dadurch verursachten Bluthochdruck, vorzugsweise von größer als 95/160 mm Hg, aufweist.

5. Verfahren zur Erzeugung einer transgenen Ratte nach einem der Ansprüche 1 bis 4, **dadurch gekennzeichnet, daß** die Gene spätestens im Achtzellenstadium in die Ratte eingeschleust werden oder in ihre Vorfahren eingeschleust worden sind.

6. Verwendung einer transgenen Ratte nach einem der Ansprüche 1 bis 4 für pharmakologische Untersuchungen zur Blutdruckregulierung.

## Claims

1. Transgenic rat, wherein its genome contains the two expressible human genes Renin and Angiotensinogen.

2. Transgenic rat according to Claim 1, wherein at least one of the genes is under the control of a homologous promoter.

3. Transgenic rat according to Claim 1, wherein at least one of the genes is under the control of a heterologous promoter.

4. Transgenic rat according to one of the Claims 1 to 3 manifesting an increased blood pressure caused by the introduced genes, preferably of greater than 90/140 mm Hg, or a high blood pressure induced thereby, preferably of greater than 95/160 mm Hg.

5. Method for the production of a transgenic rat according to one of the Claims 1 to 4, wherein the genes are introduced into the rat by no later than the eight-cell stage or have been introduced into their predecessors.

6. Use of a transgenic rat according to one of the Claims 1 to 4 for pharmacological examinations for regulation of blood pressure.

## Revendications

1. Rat transgénique se caractérisant par le fait que son génome contient les deux gènes humains exprimables, le gène de la rénine et le gène de l'angiotensinogène.

2. Rat transgénique selon la revendication 1, se caractérisant par le fait que au moins l'un de ces gènes se trouve sous le contrôle d'un promoteur homologue.

3. Rat transgénique selon la revendication 1, se caractérisant par le fait que au moins l'un de ces gènes se trouve sous le contrôle d'un promoteur hétérologue.

4. Rat transgénique selon l'une des revendications 1 à 3 qui présente une augmentation de la pression artérielle due aux gènes introduits, pression artérielle de préférence supérieure à 90/140 mm Hg, ou une hypertension due à ceci, de préférence supérieure à 95/160 mm Hg.

5. Procédé de génération d'un rat transgénique selon l'une des revendications 1 à 4, se caractérisant par le fait que les gènes seront transférés dans le rat au plus tard au stade de la division cellulaire de huit cellules ou que les gènes auront été transférés dans leurs ascendants.

6. Emploi d'un rat transgénique selon l'une des revendications 1 à 4 pour effectuer des examens pharmacologiques sur la régulation de la pression artérielle.
